# EUROPÄISCHE PATENTSCHRIFT

(11) **EP 4 049 593 B1**
(45) Veröffentlichungstag und Bekanntmachung des Hinweises auf die Patenterteilung: **23.04.2025**
(21) Anmeldenummer: 21159686.1
(22) Anmeldetag: 26.02.2021
(51) Int. Cl.: A61B 17/00

(54) **BERGEEINRICHTUNG ZUM BERGEN VON GEWEBE**
RECOVERY DEVICE FOR RECOVERING TISSUE
ÉQUIPEMENT DE CONSERVATION DES TISSUS

(43) Veröffentlichungstag der Anmeldung: 31.08.2022
(73) Patentinhaber: Erbe Elektromedizin GmbH, 72072 Tübingen (DE)
(72) Erfinder: Schnitzler, Uwe, 72074 Tübingen (DE); Enderle, Markus, 72070 Tübingen (DE)
(74) Vertreter: Rüger Abel Patentanwälte PartGmbB

(56) Entgegenhaltungen:
- WO-A1-2011/049918
- US-A- 5 190 542
- US-A1- 2001 002 437
- US-A1- 2013 023 895
- US-A1- 2020 060 665

## Beschreibung

Die Erfindung betrifft eine Bergeeinrichtung zum Bergen von Gewebe aus einem menschlichen oder tierischen Patienten, insbesondere beispielsweise aus flüssigkeitsgefüllten Körperhöhlen, wie z.B. der Harnblase.

Vorrichtungen und Einrichtungen zur Bergung von Geweben, die während eines chirurgischen Eingriffs an einem Patienten aus dem Gewebezusammenhang gelöst worden sind, sind beispielsweise aus der DE 699 07 796 T2 bekannt. Das Instrument weist ein Einführungsrohr auf, durch das sich ein axial bewegliches Stößelrohr erstreckt. Dieses ist an seinem distalen Ende mit bogenförmigen Halteschienen verbunden, an denen ein Beutel hängt. Ein durch den oberen Rand des Beutels gezogener Faden gestattet das Schließen desselben.

Aus der US 2001/0002437 A1 ist ein Bergebeutel bekannt, der aus transparentem Kunststoff, flexiblen Gestrick, Netztuch oder lose gewebtem Material bestehen kann. Dieser Bergebeutel eignet sich für den laparoskopischen Einsatz, indem er in zusammengelegter Form mittels eines Stabs durch einen entsprechenden Kanal eines Laparoskops in eine Körperhöhle eingeführt, dort entfaltet und geöffnet und mit zu entfernendem Gewebe gefüllt wird. Er kann dann geschlossen und durch das Laparoskop wieder aus der Körperhöhle gezogen werden.

Aus der US 2020/0060665 A1 ist ebenfalls ein Bergebeutel bekannt, der ein Filter aus einem schwammartigem Material aufweist. Dagegen offenbart die WO 2011/049918 A1 einen Bergebeutel aus Maschenware oder einen Mix aus Maschenware und Folie.

Weitere Bergevorrichtungen mit Beutel sind aus der WO 93/15671, der US 6,350,267 B1, der EP 2 353 511 A1, der US 2012/0158010 A1, der US 6,228,095 B1, der US 5,341,815, der EP 2 605 709 A2,der WO 2012/026809 A2, der EP 2 497 429 A1, der US 2013/0023895 A1 sowie der US 5,109,542 bekannt. Die letztgenannte Schrift offenbart einen Bergebeutel, der mit einem Gasauslass versehen ist. Als solcher ist ein hydrophobes Luft filterndes Material oder eine solche Membran vorgesehen. Das hydrophobe Filtermaterial verhindert durch seine wasserabstoßende Wirkung einen Flüssigkeitsdurchtritt, lässt jedoch Gas ausströmen.

Bei der Geweberesektion in flüssigkeitsgefüllten Körperhöhlen, beispielsweise der Harnblase, kann es darauf ankommen, krankhaft verändertes Gewebe, beispielsweise malignes Gewebe, möglichst als großes unzerteiltes Stück restlos aufzunehmen und aus der Körperhöhle zu entfernen, ohne einzelne Gewebefetzen oder Zellen zurückzulassen. Entsprechend großzügig müssen etwaige Bergebeutel bemessen sein. Andererseits müssen solche Bergebeutel durch Zugangswege, beispielsweise den Arbeitskanal eines Endoskops, aus dem Patienten entfernt werden.

Davon ausgehend ist es Aufgabe der Erfindung, eine Bergeeinrichtung und ein Verfahren zum Bergen von Gewebe zu schaffen, mit dem sich Gewebe sicher aufnehmen und mittels dessen das Gewebe über den Arbeitskanal eines Zugangsinstruments, wie beispielsweise eines Endoskops, aus dem Patientenkörper sicher entfernbar ist.

Diese Aufgabe wird mit den Merkmalen des Anspruchs 1 gelöst:

Die erfindungsgemäße Bergeeinrichtung umfasst einen Bergebeutel zum Bergen von Zellen aus einem körperinternen flüssigkeitsgefüllten Hohlraum eines Patienten. Die Flüssigkeit kann eine körpereigene Flüssigkeit, z.B. Urin, Gallenflüssigkeit, Pankreassekret, Liquor, oder auch eine extern zugeführte Flüssigkeit, z.B. Natriumchlorid-Lösung, sein. Der Bergebeutel weist eine Einfüllöffnung und ein biegesteifes oder biegeschlaffes Zugmittel auf, das mit dem Bergebeutel verbunden ist. Weiter weist der Bergebeutel wenigstens einen Abschnitt aus einer hydrophilen wasserdurchlässigen Filtermembran auf. Unter einer solchen Filtermembran wird jede Filtermembran verstanden, deren Porenweite so gering ist, dass sie Gewebefetzen und Zellen des geborgenen Gewebes sicher zurückhält. Dadurch wird es möglich, den Bergebeutel, wenn er geschlossen ist, durch Herausdrücken von Wasser auf ein Volumen zu reduzieren, das nicht wesentlich größer ist als das Volumen des aufgenommenen Gewebes. Die Bergeeinrichtung eignet sich somit insbesondere zum Einsatz in flüssigkeitsgefüllten Körperhöhlen. Es wird vermieden, dass der Bergebeutel durch ein Quantum aufgenommenen Wassers unnötig großen Raumbedarf hat. Auf diese Weise kann es gelingen, auch relativ große Gewebeabschnitte im Ganzen in dem Bergebeutel unterzubringen und den Bergebeutel dennoch nach dem Herausdrängen überschüssiger Flüssigkeit durch den relativ engen Arbeitskanal eines Zugang bietenden Instruments, beispielsweise eines Endoskops oder Zystoskops, aus dem Patienten zu entfernen.

Insbesondere bei Anwendung eines Zystoskops, das typischerweise nur einen Arbeitskanal aufweist, ist die erfindungsgemäße Bergeeinrichtung von großem Vorteil. Durch die Möglichkeit, überschüssige Flüssigkeit aus dem Bergebeutel zu entfernen und somit dessen Volumen zu reduzieren, kann der Bergebeutel große Gewebestücke aufnehmen, so dass Tumore im Ganzen entfernt werden können, ohne sie zerteilen zu müssen. Dies enthebt den Operateur auch der Notwendigkeit des mehrmaligen Wechsels zwischen Instrument und Bergeeinrichtung.

Die Einfüllöffnung des Bergebeutels kann mit einer Verschlusseinrichtung versehen sein, die zum Beispiel durch einen Profilleistenverschluss gebildet sein kann. Mit diesem ist ein fluiddichter Verschluss des Bergebeutels möglich. Der Profilleistenverschluss weist zwei zueinander komplementäre Leisten auf, die entlang ihrer gesamten Länge miteinander verrastbar sind und dabei einen fluiddichten Abschluss der Einfüllöffnung des Bergebeutels erbringen. Dazu können die beiden Leisten des Profilleistenverschlusses am proximalen Ende und am distalen Ende dicht und unlösbar verbunden sein, um im geschlossenen Zustand keine Flüssigkeit auszulassen, die irgendwelche Zellen enthalten könnte.

Die Verschlusseinrichtung weist eine Offenstellung auf, in der der Bergebeutel weit offen steht und Gewebe sowie auch Flüssigkeit aufnehmen kann. Weiter weist die Verschlusseinrichtung eine Schließstellung auf, in der die Aufnahmeöffnung des Bergebeutels geschlossen ist. Vorzugsweise ist die Verschlusseinrichtung in Richtung auf ihre Offenstellung hin vorgespannt. Die beiden Leisten des Profilleistenverschlusses sind federnd voneinander weg gewölbt.

Der Bergebeutel kann zwei vorzugsweise flexible Seitenwände aufweisen, die, wenn der Bergebeutel geschlossen ist, aufeinander liegen. Dies hilft beim Herausdrängen von Flüssigkeit, insbesondere Wasser aus dem Bergebeutel. Die Flexibilität der Seitenwände ist vorzugsweise so hoch, dass sich der Bergebeutel zu einem Wickel zusammenlegen lässt, der durch den Arbeitskanal eines Endoskops oder Zystoskops passt.

Dem Bergebeutel kann eine Auswringeinrichtung zugeordnet sein. Diese kann dazu benutzt werden, vom Bergebeutel aufgenommene Flüssigkeit, insbesondere Wasser, aus dem Bergebeutel heraus zu drängen, nachdem dieser geschlossen worden ist. Die hydrophile Filtermembran lässt Wasser und wässrige Lösungen austreten, hält jedoch Zellen und Gewebeteile zurück.

Die erfindungsgemäße hydrophile wasserdurchlässige Filtermembrane umfasst eine Anordnung aus einer oder mehrerer Lagen gleichen oder unterschiedlichen Filtermaterials.

Der Bergebeutel kann insgesamt aus dem hydrophilen wasserdurchlässigen Filtermaterial gefertigt sein. Er kann jedoch auch zumindest einen nicht wasserdurchlässigen Abschnitt aufweisen, beispielsweise in Gestalt einer Kunststofffolie, aus der eine oder beide Seitenwände des Bergebeutels ausgebildet sind. Die Kunststofffolie kann an einer oder an mehreren Stellen Fenster aufweisen, in oder an denen die hydrophile wasserdurchlässige Filtermembran angebracht ist.

Das erfindungsgemäße Filtermaterial ist ein Vlies oder eine poröse Folie.

Das Filtermaterial ist vorzugsweise ein Gewebe, ein Geflecht oder ein Gestrick.

Das Filtermaterial kann dabei aus der Gruppe folgender Stoffe ausgewählt sein:
Polyethersulfon (PES) mit oder ohne Stützmaterial, z.B. Polyester, Acrylpolymer, Polyamid (PA) oder Polyethylenterephthalat, Polysulfon (PS), Polyethylenterephtalat hydrophiliertes Polytetrafluorethylen (PTFE), Zelluloseacetat (CA), Zellulosenitrat (CN), Zelluloseacetat mit Zellulosenitrat (MCE), Nylon (NY), Polycarbonat (PCTE), Polyvinylidendifluorid (PVDF). Außerdem kann das Filtermaterial aus beschichteten Fasern oder mikroperforierten Folien, z.B. aus Zellulosefasern bestehen, beispielsweise einem papierartigen Vlies aus Zellulosefasern, das mit einer Imprägnierung zum Beispiel aus 2,2-Dimethoxy-2-phenylacetophenon sowie 3-(1H,1H,2H,2H-perfluorooctyl)-1-vinylimidazoliumiodid und 3,3'-(hexan)-1,6-diyl)bis(1-vinylimidazolium)dibromid sowie Acetonitril, Propan-1-ol beschichtet sein kann. Als besonders geeignet haben sich davon die folgenden Materialien herausgestellt: Polyethersulfon (PES), Acrylpolymer, Polyamid (PA) und Polyethylenterephthalat.

Das erfindungsgemäße Filtermaterial weist eine höchste Porengröße von kleiner als 5 µm auf. Kleinere Obergrenzen für die Porengröße können vorgesehen sein, wie beispielsweise 3 µm, 2 µm, 1,2 µm, 0,8 µm, 0,65 µm, 0,45 µm oder 0,2 µm. Obwohl prinzipiell sehr kleine Porengrößen nutzbar sind, wird auch in Betracht gezogen, die kleinste Porengröße nicht kleiner als 2 µm festzulegen, sodass sich eine hohe Wasserdurchlässigkeit der Filtermembran ergibt, zugleich aber typische Tumorzellen sicher zurückgehalten werden.

Mit der insoweit beschriebenen Bergeeinrichtung lässt sich ein Bergeverfahren wie folgt durchführen:
Zunächst wird der Bergebeutel beispielsweise durch den Arbeitskanal eines Endoskops oder Zystoskops in zusammengefaltetem oder zusammengerolltem Zustand in eine Körperhöhle eingebracht und dort freigegeben, sodass er zur Aufnahme von Zellmaterial bereit steht. Nachdem zu bergendes Material in den Bergebeutel eingebracht worden ist, wird der Bergebeutel verschlossen. Nachfolgend wird zur Volumenreduktion des Bergebeutels in diesen eingeschlossene Flüssigkeit durch die Filtermembran aus dem Bergebeutel entfernt. Der so in seinem Volumen reduzierte Bergebeutel kann nun durch den Arbeitskanal des Endoskops oder Zystoskops oder einen anderen Zugang aus der Körperhöhle eines Patienten wieder entfernt werden.

Einzelheiten und Weiterbildungen der Erfindung gehen aus den einzelnen Figuren der Zeichnung, der zugehörigen Beschreibung sowie aus Unteransprüchen hervor. Es zeigen:
Figur 1 das distale Ende eines Zystoskops mit einer darin gelagerten Bergeeinrichtung mit einem Bergebeutel, in perspektivischer Darstellung,
Figur 2 den Bergebeutel nach Figur 1, in offenem Zustand, bereit zur Aufnahme von Zellmaterial, in perspektivischer Darstellung,
Figur 3 eine ausschnittsweise Darstellung des oberen Rands des Bergebeutels, in perspektivischer geschnittener Darstellung zur Veranschaulichung seiner Verschlusseinrichtung,
Figur 4 die Verschlusseinrichtung nach Figur 3, in geschlossenem Zustand, im Vertikalschnitt,
Figur 5 einen Ausschnitt der Wand des Beutels mit Filtermembran,
Figur 6 eine abgewandelte Ausführungsform der Wand des Bergebeutels mit Filtermembran,
Figur 7 eine Bergeeinrichtung mit Auswringeinrichtung für den Bergebeutel.

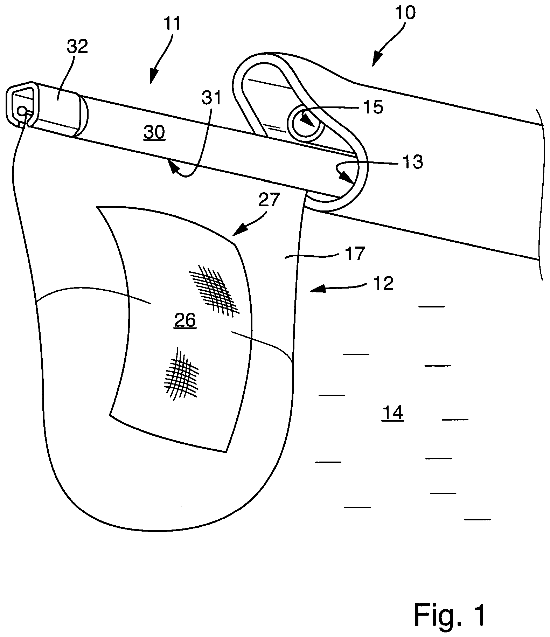

In Figur 1 ist eine in ein Zystoskop 10 eingesetzte Bergeeinrichtung 11 veranschaulicht, zu der ein Bergebeutel 12 gehört. Die Bergeeinrichtung 11 ist über einen Arbeitskanal 13 des Zystoskops 10 in eine Körperhöhle 14 eines Patienten einführbar, wie beispielsweise in eine Harnblase oder in andere Körperhöhlen in denen eine chirurgische Maßnahme wie beispielsweise eine Tumorresektion oder eine anderweitige Geweberesektion durchzuführen ist. Die Körperhöhle 14 ist dabei typischerweise mit Flüssigkeit, wie beispielsweise einer körpereigenen Flüssigkeit, z.B. Urin, Gallenflüssigkeit, Pankreassekret, Liquor, oder auch einer zugeführten Flüssigkeit, z.B. Natriumchlorid-Lösung, gefüllt. Das Zystoskop 10 weist nur den Arbeitskanal 13 auf, durch den verschiedene Instrumente in die Körperhöhle 14 einführbar sind. Solche Instrumente werden im Wechsel mit der Bergeeinrichtung 11 durch den Arbeitskanal 13 geschoben. Das Zystoskop 10 weist eine Sichteinrichtung 15 auf, mittels derer der Operationsort beobachtbar ist. Die Sichteinrichtung 15 kann eine Kamera mit Optik und Beleuchtung oder eine andere bildübertragende Einrichtung sein.

Anstelle des Zystoskops 10 kann aber auch ein Endoskop Anwendung finden, das weitere Arbeitskanäle, wie beispielsweise anstelle der Sichteinrichtung 15 einen Kanal, aufweist, durch welchen die Instrumente in die Körperhöhle 14 überführbar sind, wobei, optional, zugleich die Bergeeinrichtung 11 in dem Arbeitskanal 13 liegen kann.

Solche Instrumente können beispielsweise elektrochirurgische Instrumente mit Drahtelektrode, Schlingenelektrode oder dergleichen sein, mit denen sich Gewebepartien von sonstigen Gewebeverbünden lösen lassen. Der Bergebeutel dient der Aufnahme und dem sicheren Einschluss solcher Gewebeteile, die dann in dem Bergebeutel sicher verpackt durch den Arbeitskanal 13 aus der Körperhöhle 14 herausgeführt werden können.

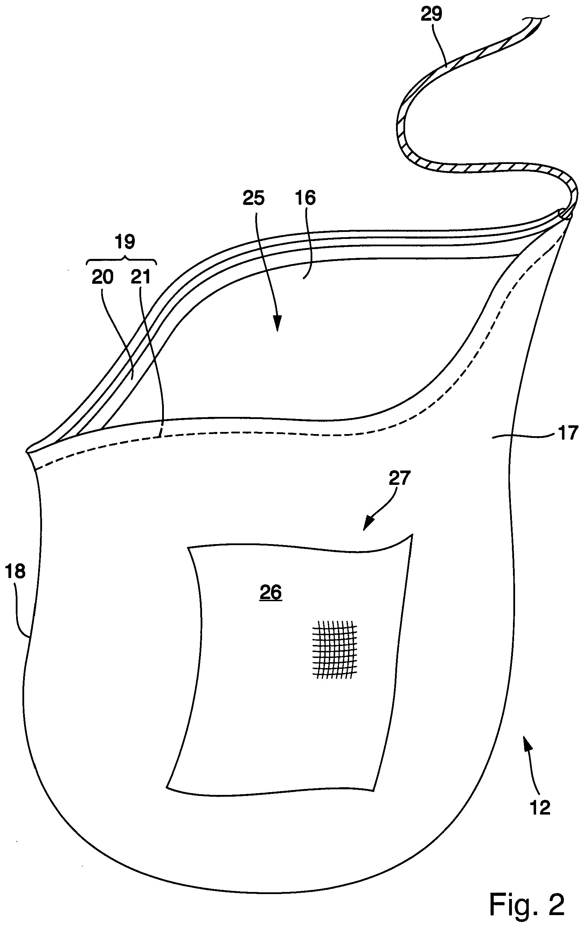

Der Bergebeutel 12 ist in Figur 2 gesondert veranschaulicht. Er weist vorzugsweise zwei flache Seitenwände 16, 17 auf, die zum Beispiel aus einem dünnen flexiblen Kunststoffmaterial bestehen können, beispielsweise einer Kunststofffolie, zum Beispiel aus Polyethylen oder einem ähnlichen Material. Die Seitenwände 16, 17 sind so geschnitten und gehen an einem Rand 18 ineinander über oder sind dort untereinander verbunden, dass sie flach aufeinander legbar sind.

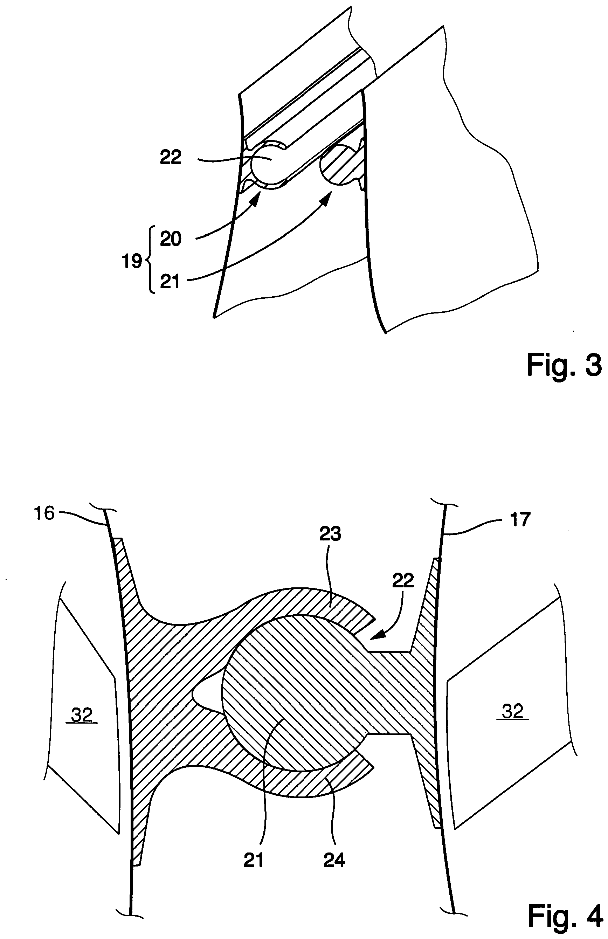

Die Seitenwände 16, 17 sind oben mit einer Verschlusseinrichtung 19 verbunden, die zum Beispiel als Profilleistenverschluss ausgebildet ist und dazu zwei komplementäre Leisten 20, 21 aufweist. Die Leiste 20 ist, wie Figur 3 zeigt, zum Beispiel im Querschnitt C-förmig und weist somit zwei federnde Schenkel auf, die zwischen einander einen Kanal 22 begrenzen. Die Leiste 21 ist vorzugsweise durch ein Rundprofil gebildet, das, wie Figur 4 zeigt, in den Kanal 22 zwischen die federnden Schenkel 23, 24 passt.

Die Leisten 20, 21 der Verschlusseinrichtung 19 sind vorzugsweise aus einem flexiblen Kunststoff ausgebildet. Wie Figur 2 zeigt, sind sie in entspannter Stellung leicht gewölbt, sodass sie an dem Bergebeutel 12 eine Einfüllöffnung 25 freigeben. Figur 2 veranschaulicht die Einfüllöffnung 25 in Offenstellung. Wenn die beiden Leisten 20, 21 jedoch auf ihrer jeweiligen ganzen Länge ineinandergreifen ist der Bergebeutel 12 geschlossen, die Verschlusseinrichtung 19 ist dann in Schließstellung. Durch die Wölbung der Leisten 20, 21 in entspanntem Zustand ist die Verschlusseinrichtung 19 jedoch, wie Figur 2 erkennen lässt, in Richtung auf ihre Offenstellung hin vorgespannt. Wird sie hingegen geschlossen, führt eine gegebenenfalls vorgesehene Steifigkeit der Seitenwände 16, 17 jedoch dazu, dass diese dazu neigen, sich aneinander anzulegen, wodurch der vom Bergebeutel 12 umschlossene Innenraum die Tendenz hat, sich zu verkleinern.

Der Bergebeutel 12 kann ganz oder wie es die Figuren 1 und 2 andeuten, auch lediglich teilweise aus einer hydrophilen wasserdurchlässigen Filtermembran 26 bestehen, die einen Abschnitt 27 des Bergebeutels 12 bzw. seiner Seitenwand 17 einnimmt. Eine gleiche oder ähnliche Filtermembran 26 kann bedarfsweise auch an der anderen Seitenwand 16 vorgesehen sein.

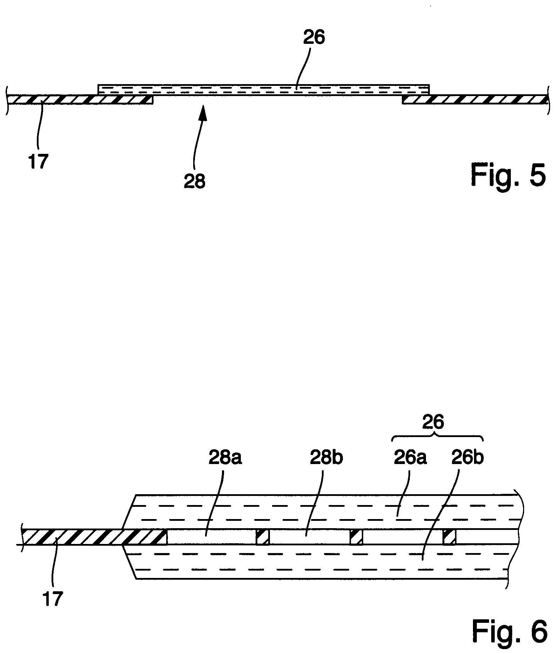

Die Filtermembran 26 ist in Figur 5 gesondert veranschaulicht. Sie ist an einem Fenster 28 der ansonsten vorzugsweise wasserundurchlässigen Seitenwand 16 angeordnet und mit dem Rand des Fensters 28 fest verbunden. Beispielsweise kann die Filtermembran an der dem Innenraum zugewandten Seite der Seitenwand 16 oder 17 angeordnet und mit dem Rand des Fensters 28 verklebt oder verschweißt sein.

Die Filtermembran 26 kann ein Vlies oder eine poröse Folie sein und einen oder mehrere aus der nachfolgenden Gruppe ausgewählten Stoffe enthalten oder aus einem solchen bestehen:

Polyethersulfon (PES), Acrylpolymer, Polyamid (PA) oder Polyethylenterephthalat, Polysulfon (PS), hydrophiliertes Polytetrafluorethylen (PTFE), Zelluloseacetat (CA), Zellulosenitrat (CN), Zelluloseacetat mit Zellulosenitrat (MCE), Nylon (NY), Polycarbonat (PCTE) und/oder Polyvinylidendiflorid (PVDF).

Alternative Filtermaterialien sind möglich, sofern sie aus einem hydrophilen Material oder aus einem Material mit hydrophiler Beschichtung oder Ausrüstung gebildet sind. Das Filtermaterial ist von Poren durchsetzt, die von einer Flachseite der Filtermembran zu ihrer anderen Flachseite führen und somit einen Fluiddurchgang bilden, durch den Wasser aus dem Innenraum des Bergebeutels 12 in die Umgebung austreten kann. Die Obergrenze für die Größe der Poren liegt vorzugsweise unter 12 µm und somit unterhalb der Zellgröße von Tumorzellen. Vorzugsweise liegt der größte Porendurchmesser bei höchstens 5 µm, weiter vorzugsweise bei höchstens 3 µm oder weniger. Vorzugsweise unterschreitet die Porengröße einen Wert von 0,2 µm nur wenig. Bei besonders bevorzugten Ausführungsformen ist der Porendurchmesser größer als 0,2 µm.

Der Bergebeutel 12 ist, wie Figur 2 erkennen lässt, mit einem Zugmittel 29, beispielsweise einem Kunststoffdraht oder einem Seil verbunden, das vorzugsweise mit einer Ecke des Bergebeutels 12 verbunden ist, an dem die Verschlusseinrichtung 19 endet. Das Zugmittel 29 weist eine Länge auf, die größer ist als die Länge des Endoskops oder Zystoskops 10, sodass das Ende des Zugmittels 29 des Bergebeutels 12 nach Figur 1 von außen (außerhalb des Patienten) her zugänglich ist. Das Zugmittel 29 erstreckt sich durch eine Schubstange 30, die wie ein Rohr ausgebildet sein kann und an ihrem distalen Ende einen Längsschlitz 31 aufweist. Der Längsschlitz ist dabei so bemessen, dass die Verschlusseinrichtung 19 geschlossen, d.h. die Leisten 20, 21 rastend ineinander gedrückt werden, wenn der Bergebeutel durch eine Relativbewegung zwischen der Schubstange 30 und dem Zugmittel 29 in den Längsschlitz 31 gezogen wird. Vorzugsweise ist das Zugmittel 29 biegeschlaff ausgebildet und kann so Zugkräfte, nicht aber Schubkräfte übertragen.

Die insoweit beschriebene Bergeeinrichtung arbeitet wie folgt:
Zur Aufnahme von Gewebe kann der Bergebeutel 12 zunächst zusammengerollt und vor oder in einem Ende 32 der Schubstange 30 positioniert werden, wobei sich das Zugmittel 29 durch die Schubstange 30 oder an dieser entlang erstreckt. Mittels der Schubstange 30 wird nun der zusammengerollte Bergebeutel 12 durch den Arbeitskanal 13 des Endoskops oder Zystoskops 10 in die Körperhöhle 14 eines Patienten geschoben. Der Bergebeutel 12 wird beim Austritt aus dem Arbeitskanal 13 des Endoskops oder Zystoskops 10 freigegeben, sodass seine Einfüllöffnung 25 den Innenraum des Bergebeutels 12 freigibt. Die Leisten 20, 21 federn voneinander weg, sodass auch zwischen den Seitenwänden 16, 17 ein Raum aufgespannt wird. Der Operateur kann nun beispielsweise mit einem anderen, durch den anderen Arbeitskanal 15 geführten Instrument Gewebe am Stück von einem Operationsort abtragen und durch die Einfüllöffnung 25 in den Bergebeutel 12 überführen. Ist dieser Arbeitsgang beendet, wird die Schubstange 30 in distaler Richtung bewegt, wobei das Zugmittel 29 axial festgehalten bleibt, sodass der Beutel 12 der Axialbewegung der Schubstange 30 nicht folgen kann. Infolgedessen schiebt sich die Schubstange 30 mit ihrem Längsschlitz 31 über das obere Ende des Bergebeutels 12 und schließt damit die Verschlusseinrichtung 19 dauerhaft. Der verschlossene Bergebeutel 12 bleibt verschlossen und lässt sich innerhalb der Körperhöhle 14 nicht, auch nicht versehentlich, öffnen. Das Zugmittel 29 kann den Bergebeutel 12 zwar in den Längsschlitz 31 hinein ziehen, aber nicht mehr heraus schieben. Wird das proximale Ende des Zugmittels 29 mit dem proximalen Ende der Schubstange 30 verbunden, kann der Bergebeutel auch nicht aus dem Längsschlitz 31 heraus geschoben werden, wenn er mittels der Schubstange 30 durch den Arbeitskanal 13 aus dem Zystoskop 10 oder Endoskop heraus gezogen wird. Dazu können an den proximalen Enden der Schubstange 30 und des Zugmittels Verbindungsmittel vorgesehen sein.

Aufgrund der Steifigkeit der Seitenwände 16, 17 und/oder auch der Steifigkeit der Filtermembran 26 hat der Innenraum des Bergebeutels 12 nun die Tendenz sein Volumen zu verringern. Im Bergebeutel 12 vorhandene, aus der Körperhöhle 14 aufgenommene Flüssigkeit, insbesondere Wasser oder wässrige Substanzen, können durch die hydrophile Filtermembran 26 aus dem Bergebeutel 12 austreten. Das aufgenommene Gewebe verbleibt jedoch in dem Bergebeutel 12. Dieser kann nun vorsichtig verkleinert, zum Beispiel zusammengerollt werden, indem er durch Drehung der Schubstange 30 um deren Ende gewickelt wird und durch den Arbeitskanal 13 zurückgezogen wird. Er kann auch mit samt des Endoskops oder Zystoskops 10 aus dem Patienten entfernt werden.

Abwandlungen der Erfindung sind möglich. Zum Beispiel kann gemäß Figur 6 anstelle eines Fensters 28 in der Seitenwand 17 eine Anzahl kleinerer Fenster 28a, 28b usw. vorgesehen sein, die von der Filtermembran 26 überdeckt sind. Außerdem ist es möglich, anstelle einer einzigen Filtermembran 26 mehrere Filtermembranen 26a, 26b vorzusehen. Die Filtermembranen 26a, 26b können als Lagen einer Filtermembran angesehen werden, wobei die dazwischenliegende gelochte Seitenwand 17 einen Träger bildet. Alternativ können die Filtermembranen 26 nach Figur 5 oder auch die Filtermembranen 26a und /oder 26b nach Figur 6 jeweils mehrlagig aufgebaut sein. Die einzelnen Lagen können aus übereinstimmenden oder auch aus unterschiedlichen Materialien aufgebaut sein, die aus der oben genannten Materialliste ausgewählt sein können. Anderweitige funktionsähnliche Materialien sind möglich. Sie können direkt aufeinanderliegend oder mit Abstand zueinander angeordnet werden. Insbesondere können viele Membranen aus Einzelfolien mit Öffnungen vorgesehen werden, die größer sind, als die Tumorzellen, z.B. (auch deutlich) größer als 12 µm. Durch Verbinden mehrerer solcher Folien in lagenweise unterschiedlichen Ausrichtungen entsteht eine Verbund-Membrane mit kleineren Öffnungen, denn es kommt zu einer Überlagerung der Einzelfolien. Dies vereinfacht die Herstellung und mindert den Widerstand des durchströmenden Wassers.

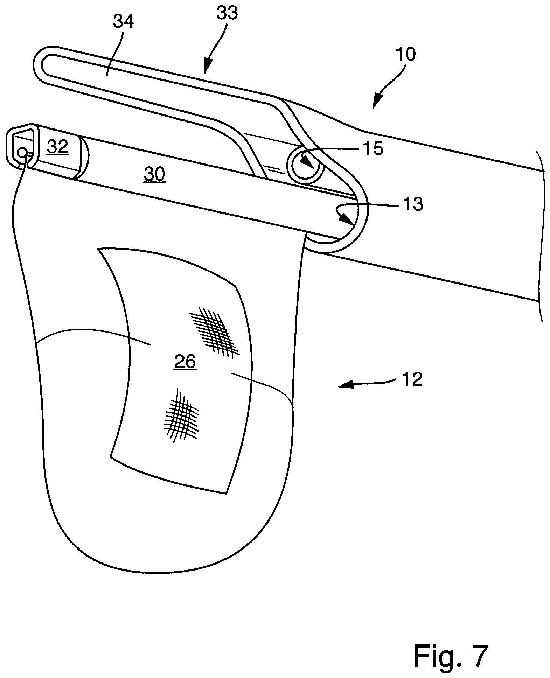

Eine weitere Ausführungsvariante zeigt Figur 7. Dort ist dem Bergebeutel 12 eine Auswringeinrichtung 33 zugeordnet. Diese kann beispielsweise als fingerartiger fester oder als Aufsatzkappe ausgebildeter Fortsatz 34 ausgebildet sein, der sich von dem Zystoskop 10 oder einem Endoskop in distaler Richtung weg erstreckt. Bei einem Endoskop kann anstelle des Fortsatzes auch eine entsprechende Stange durch den Arbeitskanal 15 gesteckt werden, deren distales Ende den Fortsatz 34 bildet bzw. ersetzt. Wird die Schubstange 30 gedreht, verhindert der Finger 34 ein Mitdrehen des Bergebeutels 12 und drückt somit Wasser aus dessen Innenraum heraus, das durch die Filtermembran 26 austreten kann.

Zum Bergen von Gewebe insbesondere von pathogenem Gewebe aus einer Körperhöhle eines Patienten kann eine Bergeeinrichtung vorgesehen sein, zu der ein Bergebeutel 12 gehört, der ganz oder teilweise aus einer hydrophilen wasserdurchlässigen Filtermembran 26 besteht. Die Filtermembran 26 weist eine Porenweite von vorzugsweise kleiner als 12 µm, weiter vorzugsweise kleiner als 8 µm und im bevorzugten Falle kleiner als 5 µm auf, um sicher den Durchtritt von pathogenen Zellen oder anderen pathogenem Material zu verhindern. Jedoch sind die Poren wenigstens so groß, dass Wasser aus dem Bergebeutel 12 leicht heraustreten kann. Auf diese Weise benötigt der Bergebeutel 12, wenn er befüllt ist, lediglich dasjenige Volumen, welches das zu entfernende, in ihm enthaltene Gewebe einnimmt. Im Bergebeutel 12 vorhandene Flüssigkeit kann durch die Filtermembran 26 aus dem Bergebeutel verdrängt werden und trägt somit nicht zu dessen Volumen bei. Dies ermöglicht die Aufnahme relativ großer Gewebestücke bei der Tumorentfernung. Es wird somit ermöglicht, Tumore im Ganzen zu entfernen, ohne sie innerhalb der Körperhöhle zerlegen zu müssen. Die Gefahr der Verschleppung von malignem Gewebe und die damit einhergehende Rezidivgefahr ist somit signifikant gesenkt.

### Bezugszeichen:

- 10: Zystoskop
- 11: Bergeeinrichtung
- 12: Bergebeutel
- 13: Arbeitskanal
- 14: Körperhöhle
- 15: Arbeitskanal / Optik
- 16, 17: Seitenwände des Bergebeutels 12
- 18: Rand
- 19: Verschlusseinrichtung
- 20: Leiste mit Schenkeln 23, 24
- 21: Leiste
- 22: Kanal
- 23: Schenkel
- 24: Schenkel
- 25: Einfüllöffnung
- 26: Filtermembrane
- 27: Abschnitt des Bergebeutels 12
- 28: Fenster
- 29: Zugmittel
- 30: Schubstange
- 31: Längsschlitz
- 32: distales Ende der Schubstange 30
- 33: Auswringeinrichtung
- 34: Finger

## Patentansprüche

1. Bergeeinrichtung (11) zum Bergen von Gewebe aus einem menschlichen oder tierischen Patienten,
mit einem Bergebeutel (12), der eine Einfüllöffnung (25) aufweist,
mit einem Zugmittel (29), das mit dem Bergebeutel (12) verbunden ist,
**dadurch gekennzeichnet, dass** der Bergebeutel (12) wenigstens einen Abschnitt (27) aus einer hydrophilen, wasserdurchlässigen Filtermembran (26) aufweist , dass die hydrophile wasserdurchlässige Filtermembrane (26) eine Anordnung aus einer oder mehreren Lagen (26a, 26b) gleichen oder unterschiedlichen Filtermaterials umfasst,
dass das Filtermaterial ein Vlies oder eine poröse Folie ist und
dass das Filtermaterial eine Porengröße von kleiner als 5 µm aufweist.

2. Bergeeinrichtung nach Anspruch 1, **dadurch gekennzeichnet, dass** die Einfüllöffnung (25) mit einer Verschlusseinrichtung (19) versehen ist.

3. Bergeeinrichtung nach Anspruch 2, **dadurch gekennzeichnet, dass** die Verschlusseinrichtung (19) ein Profilleistenverschluss (20, 21) ist.

4. Bergeeinrichtung nach Anspruch 2 oder 3, **dadurch gekennzeichnet, dass** die Verschlusseinrichtung (19) eine Offenstellung und eine Schließstellung aufweist und dass die Verschlusseinrichtung (19) in Richtung auf ihre Offenstellung vorgespannt ist.

5. Bergeeinrichtung nach einem der vorstehenden Ansprüche, **dadurch gekennzeichnet, dass** der Bergebeutel (12) zwei flache Seitenwände (16, 17) aufweist.

6. Bergeeinrichtung nach Anspruch 5, **dadurch gekennzeichnet, dass** die Seitenwände (16, 17), wenn der Bergebeutel (12) geschlossen ist, aufeinander zu gespannt sind.

7. Bergeeinrichtung nach einem der vorstehenden Ansprüche, **dadurch gekennzeichnet, dass** dem Bergebeutel (12) eine Auswringeinrichtung (33) zugeordnet ist.

8. Bergeeinrichtung nach Anspruch 2, 3 oder 4, **dadurch gekennzeichnet, dass** der Verschlusseinrichtung (19) eine Schubstange (30) mit einem Längsschlitz (31) zugeordnet ist, wobei ein die Verschlusseinrichtung (19) beherbergender Teil des Bergebeutels (12) mittels des Zugmittels (29) in den Längsschlitz einziehbar ist, um die Verschlusseinrichtung (19) zu schließen.

9. Bergeeinrichtung nach einem der vorstehenden Ansprüche, **dadurch gekennzeichnet, dass** der Bergebeutel (12) zumindest einen nicht wasserdurchlässigen Abschnitt (16, 17) aufweist.

10. Bergeeinrichtung nach Anspruch 9, **dadurch gekennzeichnet, dass** der nicht wasserdurchlässige Abschnitt (16, 17) durch eine Kunststofffolie gebildet ist.

11. Bergeeinrichtung nach Anspruch 1, **dadurch gekennzeichnet, dass** das Filtermaterial aus der Gruppe folgender Stoffe ausgewählt ist: Polyethersulfon (PES), Acrylpolymer, Polyamid oder Polyethylenterephthalat, Polysulfon (PS), hydrophiliertes Polytetrafluorethylen (PTFE), Zelluloseacetat (CA), Zellulosenitrat (CN), Zelluloseacetat mit Zellulosenitrat (MCE), Nylon (NY), Polycarbonat (PCTE), Polyvinylidendiflorid (PVDF) .

12. Bergeeinrichtung nach Anspruch 1 oder 11, **dadurch gekennzeichnet, dass** das Filtermaterial eine Porengröße von kleiner als 3 µm, kleiner als 2 µm, kleiner als 1,2 µm, kleiner als 0,8 µm, kleiner als 0,65 µm, kleiner als 0,45 µm oder kleiner als 0,2 µm aufweist.

## Claims

1. A retrieval device (11) for retrieving tissue from a human or animal patient,
having a retrieval bag (12) comprising a filling opening (25),
having a pulling means (29) connected to the retrieval bag (12),
**characterized in that** the retrieval bag (12) comprises at least one section (27) made of a hydrophilic water-permeable filter diaphragm (26),
that that the hydrophilic water-permeable filter diaphragm (26) comprises an arrangement of one or more layers (26a, 26b) of the same or different filter material,
that the filter material is a fleece or a porous film, and
that the filter material has a pore size of less than 5 µm.

2. The retrieval device according to claim 1, **characterized in that** the filling opening (25) is provided with a closure device (19).

3. The retrieval device according to claim 2, **characterized in that** the closure device (19) is a profile strip closure (20, 21).

4. The retrieval device according to claim 2 or 3, **characterized in that** the closure device (19) has an open position and a closed position and that the closure device (19) is biased toward its open position.

5. The retrieval device according to any of the preceding claims, **characterized in that** the retrieval bag (12) comprises two flat sidewalls (16, 17).

6. The retrieval device according to claim 5, **characterized in that** the sidewalls (16, 17) are biased toward one another, when the retrieval bag (12) is closed.

7. The retrieval device according to any of the preceding claims, **characterized in that** a wringing device (33) is assigned to the retrieval bag (12).

8. The retrieval device according to claims 2, 3 or 4, **characterized in that** a push rod (30) having a longitudinal slit (31) is assigned to the closure device (19), wherein a part of the retrieval bag (12) accommodating the closure device (19) can be pulled into the longitudinal slit by means of the pulling means (29) in order to close the closure device (19).

9. The retrieval device according to any of the preceding claims, **characterized in that** the retrieval bag (12) comprises at least one section (16, 17) that is not water permeable.

10. The retrieval device according to claim 9, **characterized in that** the non-water-permeable section (16, 17) is formed by a plastic foil.

11. The retrieval device according to claim 1, **characterized in that** the filter material is selected from a group of the following materials: polyethersulfone (PES), acrylic polymer, polyamide (PA) or polyethylene terephthalate, polysulfone (PS), hydrophilized polytetrafluoroethylene (PTFE), cellulose acetate (CA), cellulose nitrate (CN), cellulose acetate with cellulose nitrate (MCE), nylon (NY), polycarbonate (PCTE), polyvinylidene difluoride (PVDF)

12. The retrieval device according to claim 1 or 11, **characterized in that** the filter material has a pore size of less than 3 µm, less than 2 µm, less than 1.2 µm, less than 0.8 µm, less than 0.65 µm, less than 0.45 µm or less than 0.2 µm.

## Revendications

1. Dispositif de récupération (11) destiné à récupérer du tissu sur un patient humain ou animal,
comprenant un sachet de récupération (12) qui présente une ouverture de remplissage (25),
comprenant un moyen de traction (29) qui est relié au sachet de récupération (12),
**caractérisé en ce que** le sachet de récupération (12) présente au moins une partie (27) constituée d'une membrane filtrante (26) hydrophile perméable à l'eau,
**en ce que** la membrane filtrante (26) hydrophile perméable à l'eau comporte un ensemble formé d'une ou plusieurs couches (26a, 26b) de matériaux filtrants identiques ou différents,
**en ce que** le matériau filtrant est un non-tissé ou une feuille poreuse, et
**en ce que** le matériau filtrant présente une grosseur de pores qui est inférieure à 5 µm.

2. Dispositif de récupération selon la revendication 1, **caractérisé en ce que** l'ouverture de remplissage (25) est pourvue d'un dispositif de fermeture (19).

3. Dispositif de récupération selon la revendication 2, **caractérisé en ce que** le dispositif de fermeture (19) est une fermeture à nervures profilées (20, 21).

4. Dispositif de récupération selon la revendication 2 ou 3, **caractérisé en ce que** le dispositif de fermeture (19) présente une position ouverte et une position fermée, et **en ce que** le dispositif de fermeture (19) est mis sous précontrainte en direction de sa position ouverte.

5. Dispositif de récupération selon une des revendications précédentes, **caractérisé en ce que** le sachet de récupération (12) présente deux parois latérales (16, 17) plates.

6. Dispositif de récupération selon la revendication 5, **caractérisé en ce que** les parois latérales (16, 17) sont mises sous précontrainte l'une en direction de l'autre, lorsque le sachet de récupération (12) est fermé.

7. Dispositif de récupération selon une des revendications précédentes, **caractérisé en ce qu'**un dispositif d'essorage (33) est associé au sachet de récupération (12).

8. Dispositif de récupération selon la revendication 2, 3 ou 4, **caractérisé en ce qu'**une tige de poussée (30), dotée d'une fente longitudinale (31), est associée au dispositif de fermeture (19), sachant qu'une partie du sachet de récupération (12) qui contient le dispositif de fermeture (19) peut être rétractée dans la fente longitudinale (31) à l'aide du moyen de traction (29), en vue de fermer le dispositif de fermeture (19).

9. Dispositif de récupération selon une des revendications précédentes, **caractérisé en ce que** le sachet de récupération (12) présente au moins une partie (16, 17) non perméable à l'eau.

10. Dispositif de récupération selon la revendication 9, **caractérisé en ce que** la partie (16, 17) non perméable à l'eau est constituée d'une feuille en matière plastique.

11. Dispositif de récupération selon la revendication 1, **caractérisé en ce que** le matériau filtrant est choisi dans le groupe comprenant les matériaux suivants : polyéther sulfone (PES), polymère acrylique, polyamide ou polyéthylène téréphtalate, polysulfone (PS), polytétrafluoroéthylène (PTFE) hydrophylisé, acétate de cellulose (CA), nitrate de cellulose (CN), acétate de cellulose avec nitrate de cellulose (MCE), nylon (NY), polycarbonate (PCTE), polyfluorure de vinylidène (PVDF).

12. Dispositif de récupération selon la revendication 1 ou 11, **caractérisé en ce que** le matériau filtrant présente une grosseur de pores inférieure à 3 µm, inférieure à 2 µm, inférieure à 1,2 µm, inférieure à 0,8 µm, inférieure à 0,65 µm, inférieure à 0,45 µm ou inférieure à 0,2 µm.
